# EUROPEAN PATENT SPECIFICATION

(11) **EP 1 393 567 B1**
(45) Date of publication and mention of the grant of the patent: **31.08.2011**
(21) Application number: 02715699.1
(22) Date of filing: 16.01.2002
(51) Int. Cl.: H04N 7/18, H04N 9/47, A61B 1/04, A61B 1/06

(54) **SYSTEM AND METHOD FOR WIDE FIELD IMAGING OF BODY LUMENS**
SYSTEM UND VERFAHREN ZUR WEITWINKELABBILDUNG VON KÖRPERLUMEN
SYSTEME ET METHODE D'IMAGERIE A GRAND CHAMP DE LUMIERES CORPORELLES

(30) Priority: 16.01.2001 US 261188 P
(43) Date of publication of application: 03.03.2004
(73) Proprietor: Given Imaging Ltd., 20692 Yoqneam (IL)
(72) Inventor: MERON, Gavriel, 49556 Kfar Ganim, Petach Tikva (IL); GLUKHOVSKY, Arkady, 36790 Nesher (IL)
(74) Representative: Graf, Werner
(86) International application number: PCT/IL2002/000042
(87) International publication number: WO 2002/054932

(56) References cited:
- EP-A- 0 667 115
- US-A- 4 278 077
- US-A- 4 862 873
- US-A- 5 459 570
- US-A- 5 604 531
- US-A- 5 643 175
- US-A- 5 914 810
- US-A- 6 139 490
- US-B1- 6 240 312

## Description

### FIELD OF THE INVENTION

The present invention relates to the field of in vivo imaging. More specifically, the present invention relates to a system and method for wide angle viewing or imaging of body lumens.

### BACKGROUND OF THE INVENTION

In vivo imaging, such as the system disclosed in EP 0 667 115 A1, greatly enhances a practitioner's ability to safely and easily view internal body features and occurrences with minimal intrusion. A body lumen, typically a voluminous cavity, is most effectively viewed or imaged when a wide angle of viewing or imaging is obtained.

Wide angle optics usually employ a single optical path which includes a complicated set of alternating differently shaped mirrors and lenses. Some methods for photographing a wide field involve alignment of film and imaging devices to cover multiple fields of view.

To date, there exists no simple method or system for wide angle viewing or imaging of body lumens.

### SUMMARY OF THE INVENTION

The present invention provides a system and method for wide angle imaging of body lumens. A wide angle of imaging is obtained by employing a plurality of optical paths coordinated to cover a wider angle than that covered by a single optical path.

An optical path, in the present invention, is the course followed by light rays incident on an endo - luminal site and remitted from it onto an imager, such as a CMOS imager. Thus, an optical path, according to an embodiment of the invention, includes at least an endo- luminal site and an imager. The optical path may further include means for collecting and directing light rays, such as lenses and mirrors, for collecting the remitted light and directing and/or focusing it upon the imager.

Each optical path comprises at least one imager however, the same imager can be part of several different optical paths, where remitted light from several different endo - luminal sites is directed from the different sites onto the same imager through different optical paths.

The system according to an embodiment of the invention comprises at least one imager and an optical system having a plurality of optical paths for imaging images from within the body lumen onto the at least one imager. In one embodiment the system is inserted into the body lumen for obtaining a wide angle of view of the body lumen. The obtained images may be stored in the imager or in an additional memory device. The system may further include at least one transmitter for transmitting signals to a receiving system, such that the images can be received and analyzed in real time. The receiving system may include a recording/processing device for subsequent analysis.

The system, according to an embodiment of the invention, may be incorporated in or attached on to a device that is configured for being inserted into body lumens such as an endoscope, a needle, stent or a device that can pass through the gastrointestinal (GI) tract.

In one embodiment of the invention the system comprises a single imager and a single transmitter and a plurality of optical paths. In this embodiment a plurality of narrow field images are obtained on the single imager and are then combined into a single image having a wider angle field than any of the narrow field images.

In another embodiment of the invention the system comprises a plurality of imagers and at least one transmitter transmitting in a single channel of transmission or in multiple channels. In this embodiment the imagers are positioned such that they capture images of different portions of the body lumen. A combined image of the images captured by the different imagers shows all the individually imaged portions, thereby covering a wide field.

For example, a wide field of view of any section of the GI tract can be obtained by encapsulating the system of the invention in a device capable of passing through the entire GI tract, such as a capsule similar to the swallowable capsule described in US Patent Number 5,604,531 or WO 01/65995. US Patent Number 5,604,531 and WO 01/65995, which are assigned to the common assignee of the present application,

One imager and its optical path can be positioned at one end of the device and another imager and its optical path at another end of the device. Alternatively, imagers and their optical paths can be positioned in a circle facing outwards on the circumference of the device.

In yet another embodiment of the invention the system comprises a plurality of imagers, each having an optical path, wherein each imager and its optical path is partitioned off from its neighboring imagers. In this embodiment interference between imager operations is greatly reduced.

The present invention further provides a device for imaging a body lumen.
The device, according to an embodiment of the invention, comprises the system of the invention. In one embodiment the device can be inserted into the GI tract, for viewing specific regions of the GI tract, such as an endoscope, or for viewing the entire GI tract, such as a swallowable capsule.

The method for obtaining a wide field image of a body lumen, according to an embodiment of the invention, comprises the following steps: 1. obtaining a plurality of images from within the body lumen from the plurality of images, 2. transmitting signals from the imagers, 3. receiving the transmitted signals and 4. combining the plurality of images into a single image. The obtained images may be stored in a memory device for further analysis.

### BRIEF DESCRIPTION OF THE DRAWINGS

The present invention will be understood and appreciated more fully from the following detailed description taken in conjunction with the appended drawings in which:
Figure 1A is a schematic longitudinal cross section view of a device comprising the system comprising a single imager, according to an embodiment of the invention;
Figure 1B is a schematic radial cross section view of the device shown in Fig. 1A;
Figure 1C is a more detailed view of the imager shown in Fig. 1B;
Figure 2A is a schematic longitudinal cross section view of the system comprising five optical paths, according to an embodiment of the invention;
Figure 2B is a schematic radial cross section view of the system shown in Fig. 2A;
Figure 3 is a schematic presentation of the combining of four images to a single combined image, according to an embodiment of the invention;
Figure 4 is a schematic longitudinal cross section view of a device comprising the system that comprises front and rear optical paths, according to an embodiment of the invention;
Figure 5 is a schematic radial cross section view of the system comprising a plurality of imagers, according to an embodiment of the invention;
Figure 6 is a schematic longitudinal cross section view of a device comprising the system comprising a plurality of optical paths partitioned off from each other, according to an embodiment of the invention; and
Figure 7 is a schematic radial cross section view of a device comprising a system that comprises a plurality of optical paths partitioned off from each other, according to an embodiment of the invention.

### DETAILED DESCRIPTION OF THE INVENTION

The system and method, according to an embodiment of the present invention, enable wide angle imaging of body lumens. The wide angle of view is obtained by imaging partially overlapping or non overlapping portions of the body lumen, using a plurality of optical paths. The images of the body lumen portions may be combined to a single image.

The imaging of different portions of the body lumen, which may or may not be simultaneous, is done by employing a plurality of optical paths, each of which covers a different portion of the body lumen. In accordance with different embodiments of the invention, and as will be shown in the figures, light rays may be collimated through the different optical paths to a single imager or to a plurality of imagers. The different optical paths may or may not be separated from each other by a physical partition.

A separate transmitter and channel of transmission may be assigned to each imager for simultaneous transmitting of signals from the body lumen.

Alternatively, the output of several imagers may be combined over a single transmitter and channel of transmission e.g. by using different carrier frequencies. According to one embodiment, the combination of information from the different imagers over the single channel may be done either by selecting a bit from a different imager each time, thus transmitting all the images almost simultaneously, or by transmitting image after image.

The system according to an embodiment of the invention comprises a plurality of optical paths and at least one imager for endo-luminal imaging. In one embodiment the system may be incorporated in or attached to any device or probe suitable for being inserted into a body lumen, such as at the tip of a needle, on the inserted end of an endoscope or in a swallowable capsule. In one embodiment the system is positioned behind an optical window.

In the following description, various aspects of the present invention will be described. For purposes of explanation, specific configurations and details are set forth in order to provide a thorough understanding of the present invention. However, it will also be apparent to one skilled in the art that the present invention may be practiced without the specific details presented herein. Furthermore, well known features may be omitted or simplified in order not to obscure the present invention.

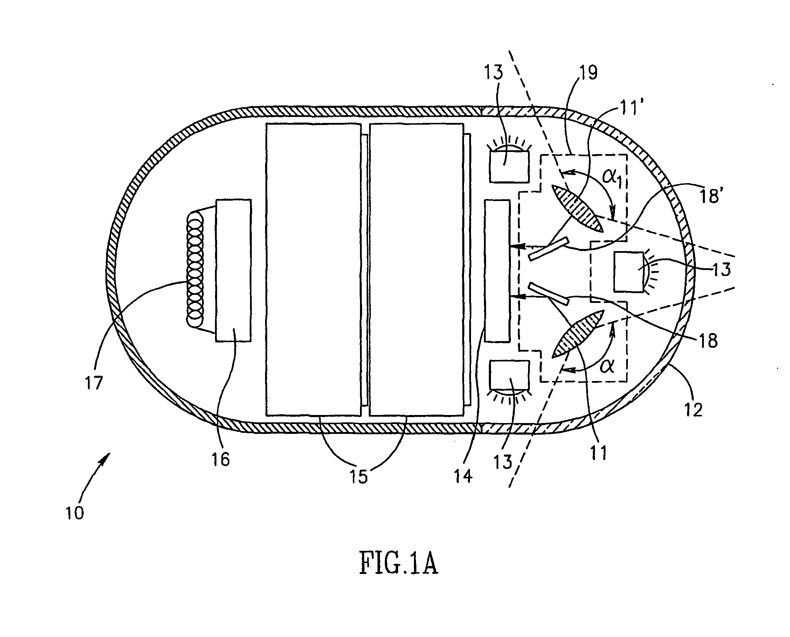

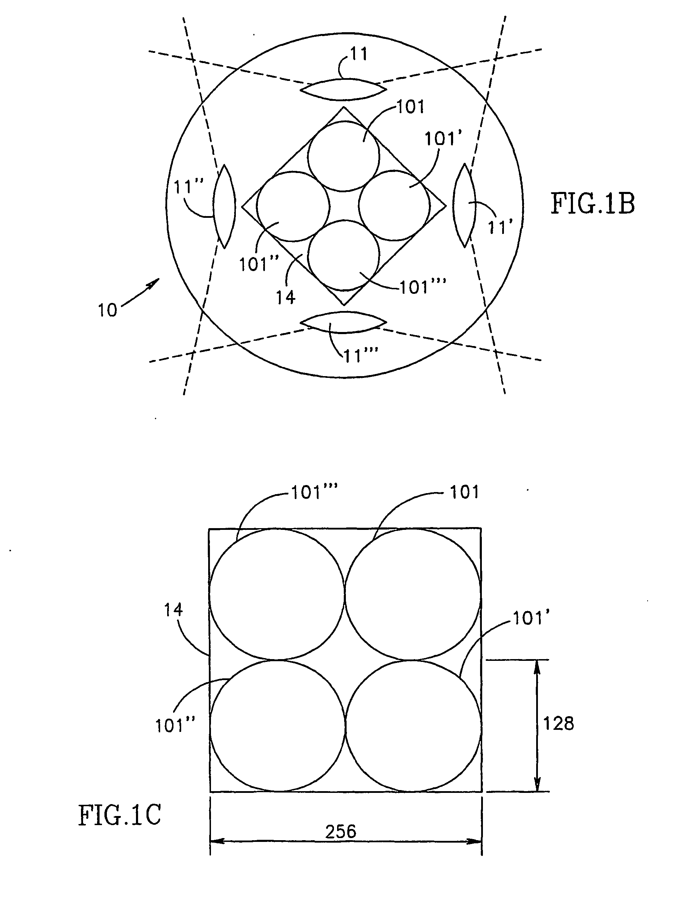

A swallowable capsule comprising a video camera for imaging the GI tract is described in the above mentioned US Patent Number 5,604,531 and WO 01/65995. A capsule that may comprise similar elements to the capsules described in US Patent Number 5,604,531 and WO 01/65995 but that comprises a plurality of optical paths, is schematically shown in Fig. 1. The capsule 10 comprises illumination sources 13, such as light emitting diods (LEDs), a system 19 including lenses 11 and mirrors 18, all positioned behind optical window 12, an imager 14, such as a CMOS imager for obtaining images from within the GI tract, power source 15, such as a battery, which provides power to the entirety of the electrical elements of the capsule and transmitter 16 and antenna 17 for transmitting video signals from imager 14. Signals may be transmitted using various digital or analog modulation techniques. For example transmission of a digital image over a radio channel may use an FSK (Frequency Shift Keying) modulation technique.

Externally to the body there is a receiver (not shown) for receiving the signals transferred by transmitter 16 and antenna17.

Light rays from illumination sources 13 illuminate the GI tract inner wall. Remitted light rays are collected by lenses 11 and 11' and directed by mirrors 18 and 18' to imager 14. Lens 11 gathers illumination rays at angle α, obtaining an image field that is defined by angle α. Lens 11' gathers illumination rays at angle α1, obtaining an image field that is defined by angle α1. Thus, by using both lenses 11 and 11', an image field defined by angle (α + α1) can be covered.

Two more optical paths can be situated in parallel to the paths which include lens 11 and mirror 18 and lens 11' and mirror 18' so that an image field twice as wide can be covered. In this manner, a wide field in different planes can be imaged, as further shown in Fig. 1B.

Fig. 1B is a schematic radial cross section illustration of a system comprising four optical paths 11, 11', 11" and 11"' in all of which remitted light is directed to a single imager 14. The four fields 101, 101', 101" and 101"' covered by the four optical paths 11, 11', 11" and 11"' are imaged onto imager 14 coincidentally. As shown in Fig. 1C each of the fields 101, 101', 101" and 101"' takes up a quarter of the imager 14 face area (for example, 128X128 pixels of the 256X256 pixels area of the imager).

The optical schemes illustrated in Figs. 1A and 1B provide a viewing angle that is larger than 180°, in the longitudinal plane (Fig 1A) and 360° imaging in a plane perpendicular to the longitudinal plane (Fig 1B).

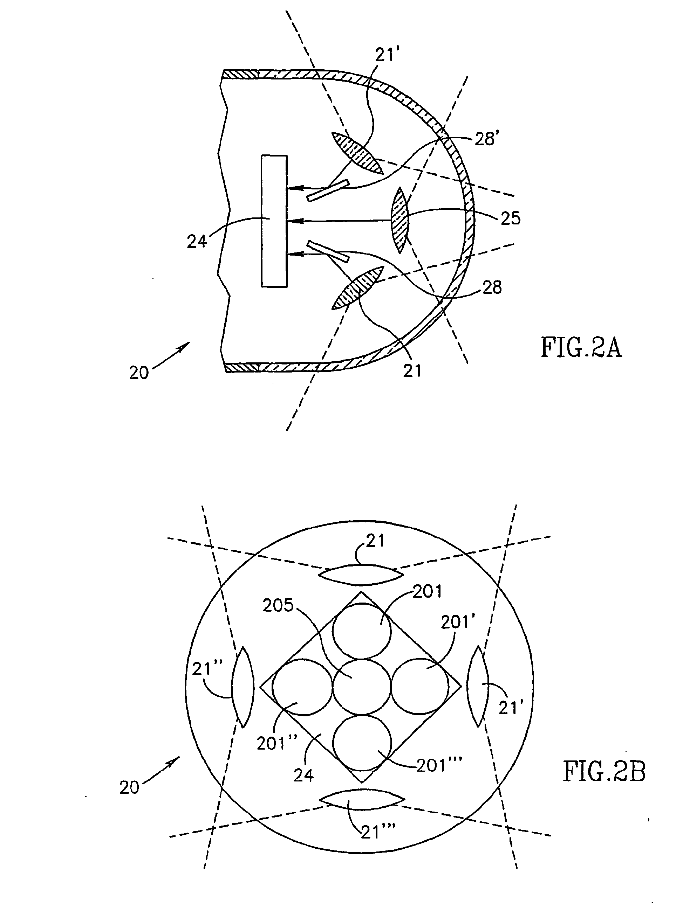

Similarly, the system of the invention may comprise five optical paths, as shown in Fig. 2A. Fig. 2A schematically illustrates the system comprising an optical path which includes lens 21 and mirror 28, an optical path which includes lens 21' and mirror 28' and an optical path which includes lens 25. Together with two more optical paths that are situated in parallel to the paths that include lens 21 and mirror 28 and lens 21' and mirror 28', there are five optical paths covering a wide image field.

Fig. 2B is a schematic radial cross section illustration of a system comprising five optical paths (only four optical paths 21, 21', 21" and 21"' are shown) in which remitted light is directed to a single imager 24. The five fields 201, 201', 201", 201"' and 205 covered by the five optical paths are imaged onto imager 24 coincidentally.

The five optical path scheme shown in Figs. 2A and 2B provides optionally better overlapping between margins of neighboring optical path fields than the four optical path scheme and thus enables better reconstruction of a single combined image.

The system illustrated in Figs. 2A and 2B can be incorporated in or attached to device 20 which may be an endoscope, a needle, a stent, a capsule designed to pass through the GI tract, etc.

The components of the system according to an embodiment of the invention may be specifically designed for the system, or the system may utilized some components from other systems that operate in body lumens, thus economically taking advantage of existing components. For example, the system of the invention may be incorporated into or affixed onto medical devices meant for being inserted into body lumens, such as needles, stents, endoscopes or capsules designed to pass through the entire GI tract. For example, endoscopes utilize a light source and sometimes an imaging device while operating. Thus, the system of the invention can be incorporated into an endoscope and utilize the endoscope's light source and imaging device.

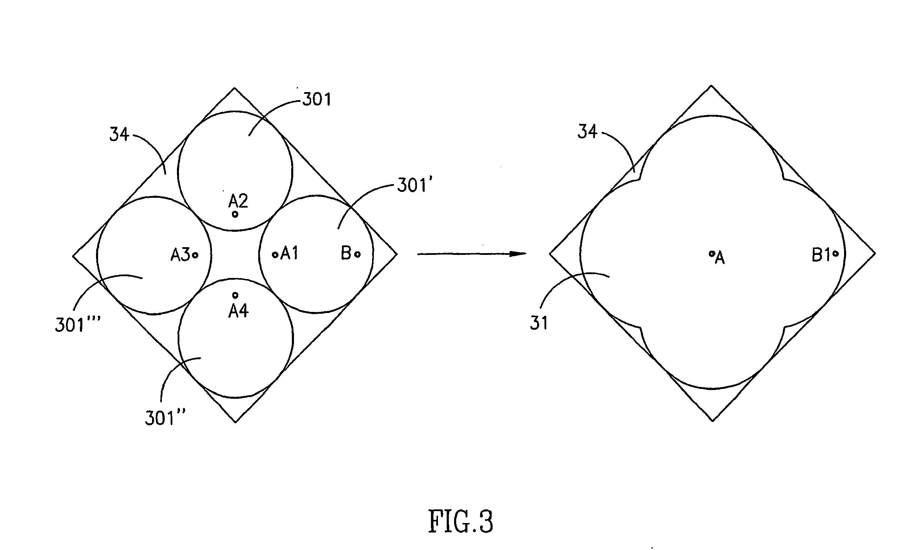

Combining multiple images to a single image is schematically shown in Fig. 3. The four different fields 301, 301', 301" and 301"' imaged on imager 34 are combined into a single image 31 which is a wide angle image of the lumen being viewed. The obtained images may be presented separately for a physician's examination, or may be combined and presented as a single wide-angle image.

As shown in Figs.1B and 2B there can be an overlap between the fields obtained by the different optical paths. Thus, part of the fields 301, 301', 301" and 301'" may be the same. For example, points A1, A2, A3 and A4 all represent the same pixel, while point B represents a pixel unique to field 301'.

The combination into the single image 31 may be performed, for example, by applying an algorithm which assigns each image point (pixel) in the different fields 301, 301', 301" and 301"' to another point (pixel) of the single image 31. Thus, points A1, A2, A3 and A4 will be assigned to point A in image 31 and point B will be assigned to point B1 in image 31.

The four different fields 301, 301', 301" and 301"' may be combined by a combiner in the receiving system which is adapted to combine a plurality of images into a single image. The operation of combining images into a single image usually requires significant processing effort and computing resources. Therefore this operation is usually performed off-line (after receiving the image transmitted from the imager) in an external recording/processing device (not shown).

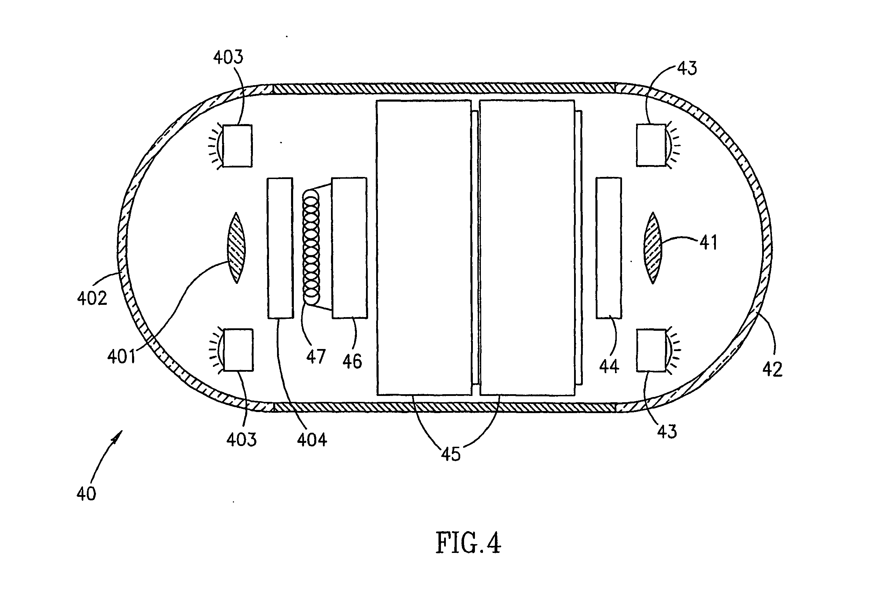

Another embodiment of the invention is schematically illustrated in Fig. 4, in which a longitudinal cross section of device 40 is schematically shown. Device 40 comprises two optical domes 42 and 402 behind which are situated illumination sources 43 and 403 and optical paths 41 and 401, respectively. In optical paths 41 and 401 remitted light is directed to imagers 44 and 404 respectively. The device 40 further comprises power source 45, which provides power to the entirety of electrical elements of the device, and transmitter 46 and antenna 47 for transmitting video signals from the imagers 44 and 404. The system of the invention, as operable in device 40, is capable of simultaneously obtaining images of the body lumen, for example, the GI tract, from two ends of the device. For example, device 40 may be a cylindrical capsule having a front end and a rear end, which is capable of passing the entire GI tract. The system in a cylindrical capsule can image the GI tract in the front and in the rear of the capsule.

In one embodiment, device 40 is inserted into a body lumen, for example a patient's GI tract. The patient may swallow the device and it will start its journey in the patient's GI tract. Some parts of this journey are through voluminous areas of the GI tract, for example, the stomach or the large intestine, in which the device 40 is not always oriented "head first", with one end of the device leading and the other end following. In the wider areas of the GI tract the device 40 may tumble in a rotating motion through the GI tract. In this case, simultaneous imaging of the front and rear of the device 40 is advantageous in imaging a wide field of the lumen. Also, there is an advantage in viewing the same site from different angles (as imaged by the front imager e.g., 44 and by the rear imager e.g., 404), especially, for example, if the site is enclosed in a fold of the GI tract.

Both images obtained by imagers 44 and 404 may be transmitted by transmitter 46 serially or simultaneously, as described above. Optionally, each imager may be assigned a separate transmitter and channel for transmitting the images. The two images (front and rear) can be displayed separately or as a single combined image.

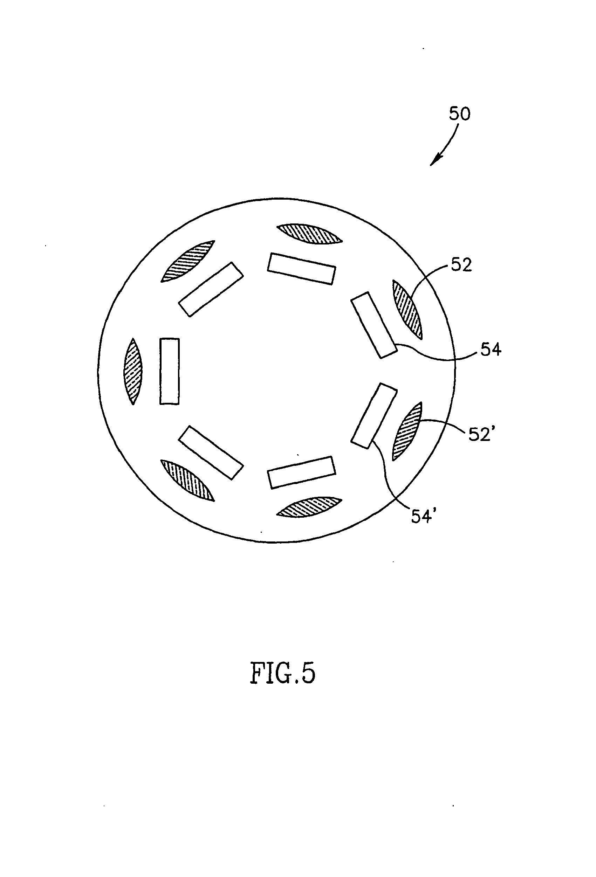

In another embodiment of the invention a plurality of optical paths and a plurality of imagers are employed. The system schematically illustrated in Fig. 5 comprises a plurality of optical paths, for example 52 and 52' having a plurality of imagers, for example 54 and 54'. In each optical path 52 and 52', which may include lenses and mirrors for gathering and collimating remitted light, the remitted light is directed to a separate imager 54 and 54' respectively, such that different portions of the body lumen are imaged on different imagers. Optical paths 52 and 52' may be situated in capsule 50, which is designed to pass through and image the entire GI tract.

The different images, which may be acquired simultaneously, can be transferred to a receiving system over a single channel or over a multi channel link, as described above.

In a single channel link the concurrently acquired images are transferred serially and a multiplexer may be used to effect transmission. A single channel link operates at a higher frequency than a multi channel link. In a multi channel link the images can be transferred in parallel and at a lower frequency and thus have larger bandwidths. The multi channel link may thus be more suitable for transferring larger amounts of data. Also, the lower frequency multi channel link may be more suitable for use with a capsule 50 designed to pass through and image the entire GI tract in which the frequency used for transmitting images from the GI tract is usually limited to the 200 - 500 MHz range.

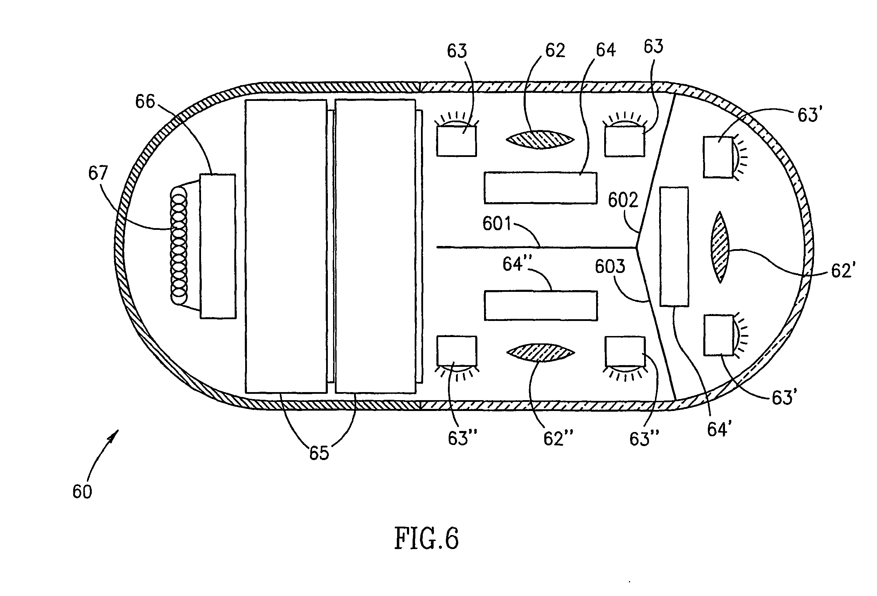

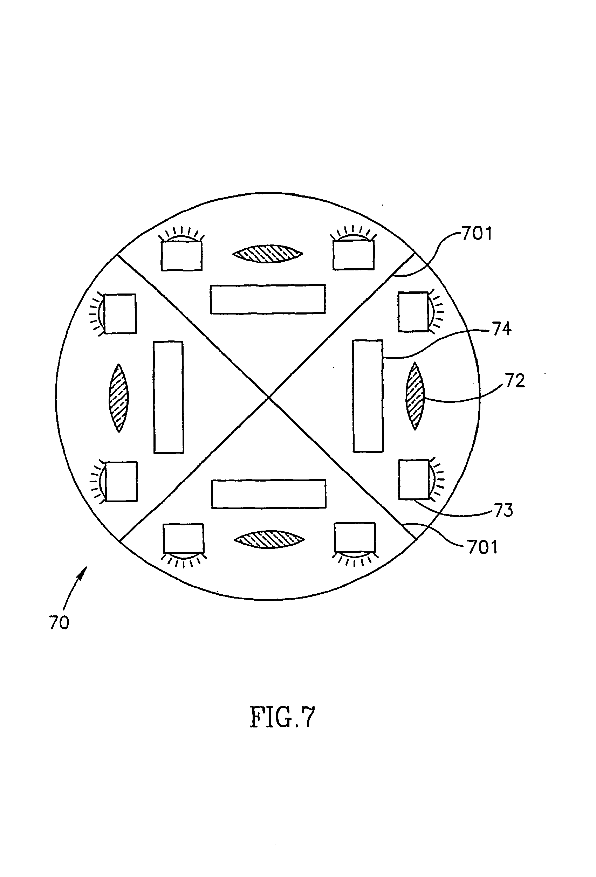

Another embodiment of the invention is schematically illustrated in Figs. 6 and 7. In Fig. 6 a longitudinal cross section of device 60 is schematically illustrated. Device 60 comprises a plurality of optical paths 62, 62' and 62" in which remitted light is directed to imagers 64, 64' and 64" respectively. Each optical path and respective imager are partitioned off from the other optical paths and imagers by partitions 601, 602 and 603, and each partitioned optical path has its own illumination source 63, 63' and 63". In one embodiment the device 60 comprises an optical dome which may extend over most of the device, covering the optical paths 62, 62' and 62", or the device may include an optical dome covering optical path 62' and optical windows on the sides of the device covering optical paths 62 and 62". Partitions 601, 602 and 603 may be made of opaque material and may serve to prevent interference between the operation of the different imagers and their associated illumination sources.

A radial cross section of a device comprising a plurality of optical paths partitioned from each other is shown in Fig. 7. The device 70 may be cylindrical shaped, similar to a swallowable capsule or to an endoscope tip, or it may a transparent sphere shaped device or it may be of any shape suitable for being inserted into and passing through a body lumen.

In device 70 each of the imagers 74 faces a portion of the body lumen at 90° to its neighboring imager. The body lumen is illuminated by illumination sources 73 and remitted light is directed onto imager 74 through optical path 72. In this manner a complete 360° field can be imaged. The mechanical dividers, partitions 701, serve to avoid having interference between the different imagers 74 and illumination sources 73.

It will be appreciated by persons skilled in the art that the present invention is not limited by what has been particularly shown and described herein above. Rather the scope of the invention is defined by the claims which follow.

## Claims

1. A system for imaging a lumen of a body comprising a receiving system external to said body and an imaging capsule (50, 60, 70) which comprises a transmitter (66) for transmitting image signals to said receiving system **characterized in that** said capsule (50, 60, 70) comprises a plurality of imagers (54, 54', 64, 64" , 74) positioned in a circle facing outwards on a circumference of the capsule (50, 60, 70) and an optical system having a plurality of lenses for obtaining a plurality of optical paths for imaging images from within the body lumen onto the plurality of imagers (54, 54', 64, 64" , 74).

2. The system according to claim 1, wherein the capsule (50, 60, 70) further comprises an optical window and/or an optical dome which is at least partially disposed on sides of the capsule (50, 60, 70), such that optical paths are covered which are perpendicular to a longitudinal axis of the capsule (50, 60, 70).

3. The system according to any of the preceding claims, wherein the transmitter (66) and the receiving system are built such that said signals are transferred serially by said transmitter (66) over a single channel.

4. The system according to claim 1 or 2 wherein the transmitter (66) transmits in multiple channels.

5. The system according to any of the preceding claims wherein at least one of the plurality of imagers (54, 54', 64, 64" , 74) further comprises a memory device for storing images.

6. The system according to any of the preceding claims wherein the receiving system comprises a recording/processing device.

7. The system according to claim 6 wherein the receiving system comprises a combiner adapted to combine a plurality of images into a single image.

8. The system according to any of the preceding claims wherein the imagers (54, 54', 64, 64" , 74) and their respective optical paths (52, 52', 62, 62" , 72) are partitioned off from each other.

9. The system according to claim 8 wherein each of the plurality of imagers (64, 64" , 74) faces a portion of the body lumen at 90° to its neighbouring imager (64, 64" , 74).

10. The system according to any of the preceding claims wherein the capsule (50, 60, 70) is configured for being inserted into a body lumen.

11. The system according to claim 10 wherein the body lumen is the gastrointestinal tract.

12. A method for operating a system according to any of the preceding claims, wherein the capsule (50, 60, 70) has been pre-inserted into the body lumen, comprising the steps of:
- obtaining images of said body lumen from the plurality of imagers (54, 54', 64, 64" , 74);
- transmitting to the receiving system signals representing said images;
- storing a plurality of said images; and
- combining the plurality of images into a single image.

13. The method of claim 12, wherein said signals are transferred serially over a single channel.

## Patentansprüche

1. System zur Abbildung eines Körperlumens umfassend ein Empfangssystem ausserhalb des genannten Körpers und eine Bild gebende Kapsel (50, 60, 70) die eine Übertragungsvorrichtung (66) zur Übertragung von Bildsignalen zum genannten Empfangssystem umfasst, **dadurch gekennzeichnet, dass** die genannte Kapsel (50, 60, 70) eine Mehrzahl von Bildaufnahmevorrichtungen (54, 54', 64, 64", 74) umfasst, die nach Aussen ausgerichtet in einem Kreis an einem Umfang der Kapsel (50, 60, 70) angeordnet sind, und ein optisches System aufweisend eine Mehrzahl von Linsen zum Erhalten einer Mehrzahl von optischen Pfaden zum Abbilden von Bildern von innerhalb des Körperlumens auf die Mehrzahl der Bildaufnahmevorrichtungen (54, 54', 64, 64", 74).

2. Das System gemäss Anspruch 1, wobei die Kapsel (50, 60, 70) zudem ein optisches Fenster und/oder einen optischen Dom umfasst, welcher zumindest teilweise an Seiten der Kapsel (50, 60, 70) angeordnet ist, so dass optische Pfade mit erfasst sind, die senkrecht zu einer longitudinalen Achse der Kapsel (50, 60, 70) sind.

3. Das System gemäss einem der vorhergehenden Ansprüche, wobei die Übertragungsvorrichtung (66) und das Emfangssystem derart ausgestaltet sind, dass die genannten Signale durch die genannte Übertragungsvorrichtung (66) seriell über einen einzigen Kanal übertragen sind.

4. Das System gemäss Anspruch 1 oder 2, wobei die Übertragungsvorrichtung (66) in mehreren Kanälen überträgt.

5. Das System gemäss einem der vorhergehenden Ansprüche, wobei zumindest eine der Mehrzahl von Bildaufnahmevorrichtungen (54, 54', 64, 64", 74) zudem eine Speichervorrichtung zum Speichern von Bildern umfasst.

6. Das System gemäss einem der vorhergehenden Ansprüche, wobei das Empfangssystem eine Aufzeichnungs-/Verarbeitungs-Vorrichtung umfasst.

7. Das System gemäss Anspruch 6, wobei das Empfangssystem eine Vereinigungsvorrichtung umfasst, die ausgestaltet ist um eine Mehrzahl von Bildern in ein einziges Bild zu vereinigen.

8. Das System gemäss einem der vorhergehenden Ansprüche, wobei die Bildaufnahmevorrichtungen (54, 54', 64, 64", 74) und deren entsprechenden optischen Pfade (52, 52', 62, 62", 72) voneinander abgetrennt sind.

9. Das System gemäss Anspruch 8, wobei jede der Mehrzahl von Bildaufnahmevorrichtungen (64, 64", 74) sich einem Teil des Körperlumens zuwendet, unter 90° zu seiner benachbarten Bildaufnahmevorrichtung (64, 64", 74).

10. Das System gemäss einem der vorhergehenden Ansprüche, wobei die Kapsel (50, 60, 70) ausgestaltet ist zum Einführung in ein Körperlumen.

11. Das System gemäss Anspruch 10, wobei das Körperlumen der Gastrointestinaltrakt ist.

12. Ein Verfahren zum Betrieb eines Systems gemäss einem der vorhergehenden Ansprüche, wobei die Kapsel (50, 60, 70) vorher eingeführt wurde in das Körperlumen, umfassend die Schritte:
- erhalten von Bildern des genannte Körperlumens von der Mehrzahl von Bildaufnahmevorrichtungen (54, 54', 64, 64", 74);
- übertragen von Signalen, welche die genannten Bilder repräsentieren, an das Empfangssystem;
- speichern einer Mehrzahl der genannten Bildern; und
- kombinieren der Mehrzahl von Bildern in ein einziges Bild.

13. Das Verfahren gemäss Anspruch 12, wobei die genannten Signale seriell über einen einzigen Kanal übertragen werden.

## Revendications

1. Système d'imagerie d'une lumière d'un corps comprenant un système de réception externe audit corps et une capsule d'imagerie (50, 60, 70) qui comprend un émetteur (66) destiné à émettre des signaux d'image vers ledit système de réception **caractérisé en ce que** ladite capsule (50, 60, 70) comprend une pluralité de modules d'imagerie (54, 54', 64, 64", 74) positionnés suivant un cercle orienté vers l'extérieur sur une circonférence de la capsule (50, 60, 70) et un système optique présentant une pluralité de lentilles destinées à obtenir une pluralité de trajets optiques pour former des images provenant de l'intérieur de la lumière corporelle sur la pluralité de modules d'imagerie (54, 54', 64, 64", 74).

2. Système selon la revendication 1, dans lequel la capsule (50, 60, 70) comprend en outre une fenêtre optique et/ou un dôme optique qui est au moins partiellement disposé sur les côtés de la capsule (50, 60, 70), de telle sorte que les trajets optiques sont couverts, lesquels sont perpendiculaires à un axe longitudinal de leur capsule (50, 60, 70).

3. Système selon l'une quelconque des revendications précédentes, dans lequel l'émetteur (66) et le système de réception sont construits de telle sorte que lesdits signaux sont transférés en série par ledit émetteur (66) sur un seul canal.

4. Système selon la revendication 1 ou 2, dans lequel l'émetteur (66) émet dans de multiples canaux.

5. Système selon l'une quelconque des revendications précédentes, dans lequel au moins un parmi la pluralité de modules d'imagerie (54, 54', 64, 64", 74) comprend en outre un dispositif à mémoire destiné à stocker des images.

6. Système selon l'une quelconque des revendications précédentes, dans lequel le système de réception comprend un dispositif d'enregistrement/de traitement.

7. Système selon la revendication 6, dans lequel le système de réception comprend un module de combinaison conçu pour combiner une pluralité d'images en une seule image.

8. Système selon l'une quelconque des revendications précédentes, dans lequel les modules d'imagerie (54, 54', 64, 64", 74) et leurs trajets optiques respectifs (52, 52', 62, 62", 72) sont séparés les uns par rapport aux autres.

9. Système selon la revendication 8, dans lequel chaque module parmi la pluralité de modules d'imagerie (64, 64", 74) fait face à une partie de la lumière corporelle à un angle de 90° par rapport à son module d'imagerie voisin (64, 64", 74).

10. Système selon l'une quelconque des revendications précédentes, dans lequel la capsule (50, 60, 70) est configurée pour être insérée dans une lumière corporelle.

11. Système selon la revendication 10, dans lequel la lumière corporelle est une voie gastro-intestinale.

12. Procédé destiné à mettre en oeuvre un système selon l'une quelconque des revendications précédentes, dans lequel la capsule (50, 60, 70) a été pré-insérée dans la lumière corporelle, comprenant les étapes consistant à :
- obtenir des images de ladite lumière corporelle à partir de la pluralité de modules d'imagerie (54, 54', 64, 64", 74),
- émettre vers le système de réception des signaux représentant lesdites images,
- mémoriser une pluralité desdites images, et
- combiner la pluralité d'images en une seule image.

13. Procédé selon la revendication 12, dans lequel lesdits signaux sont transférés en série sur un seul canal.
